# EUROPEAN PATENT SPECIFICATION

(11) **EP 0 927 052 B1**
(45) Date of publication and mention of the grant of the patent: **16.04.2003**
(21) Application number: 97938438.5
(22) Date of filing: 15.08.1997
(51) Int. Cl.: A61L 27/00, A61K 35/38, A61F 2/00

(54) **BLADDER SUBMUCOSA SEEDED WITH CELLS FOR TISSUE RECONSTRUCTION**
BLASENMUKOSA MIT ZELLEN FÜR DIE REKONSTRUKTION VON GEWEBEN
SOUS-MUQUEUSE VESICALE ENSEMENCEE PAR DES CELLULES POUR LA RECONSTITUTION DE SON TISSU

(30) Priority: 16.08.1996 US 24029 P; 16.08.1996 US 24069 P
(43) Date of publication of application: 07.07.1999
(73) Proprietor: The Children's Medical Center Corporation, Boston, Massachusetts 02115 (US)
(72) Inventor: ATALA, Anthony, Weston, MA 02193 (US)
(74) Representative: Price, Vincent Andrew
(86) International application number: US9714604
(87) International publication number: WO98006445

(56) References cited:
- WO-A-90/00395
- WO-A-93/05798
- WO-A-93/07913
- WO-A-96/31226
- WO-A-96/31232
- US-A- 5 275 826
- US-A- 5 352 463
- US-A- 5 554 389

## Description

### Background of the Invention

Reconstructive surgery has been used for many years for the treatment of congenital tissue defects and for repair of damaged organs and tissues. An ideal material for tissue reconstruction should be biocompatible, able to incorporate into the native tissue without inducing an adverse tissue response, and should have adequate anatomical and functional properties (for example, size, strength, durability, and the like). Although a large number of bio-materials, including synthetic and naturally-derived polymers, have been employed for tissue reconstruction or augmentation (see, e.g., "Textbook of Tissue Engineering" Eds. Lanza, R., Longer, R., and Chick, W., ACM Press, Colorado (1996) and references cited therein), no material has proven satisfactory for use in every application.

For example, in the field of bladder reconstruction, synthetic biomaterials such as polyvinyl and gelatin sponges, polytetrafluoroethylene (Teflon) felt, and silastic patches have been relatively unsuccessful, generally due to foreign body reactions (see, e.g., Kudish, H.G., *J. Urol*. 78:232 (1957); Ashkar, L. and Heller, E., *J. Urol*. 98:91 (1967); Kelami, A. et al., *J. Urol*. 104:693 (1970)). Polymeric materials have been used as "scaffolds" for seeding cells; the seeded scaffolds can be implanted to provide a matrix for the growth of new tissue (see, e.g., Atala, A. et al., *J. Urol*. 148 (2 Pt 2): 658-62 (1992); Atala, A., et al. *J. Urol*. 150 (2 Pt 2): 608-12 (1993)). Naturally-derived materials such as lyophilized dura, deepithelialized bowel segments, and small intestinal submucosa (SIS) have also been proposed for bladder replacement (for a general review, see Mooney, D. et al., "Tissue Engineering: Urogenital System" in "Textbook of Tissue Engineering" Eds. Lama, R., Langer, R., and Chick, W., ACM Press, Colorado (1996)).

It has been reported that bladders augmented with data, peritoneum, placenta and fascia contract over time (Kelami, A. et al., *J. Urol*. 105:518 (1971)). De-epithelized bowel segments demonstrated an adequate urothelial covering for use in bladder reconstruction, but difficulties remain with either mucosal regrowth, segment fibrosis, or both. It has been shown that de-epithelization of the intestinal segments may lead to mucosal regrowth, whereas removal of the mucosa and submucosa may lead to retraction of the intestinal segment (see, e.g., Atala, A., *J. Urol.* 156:338 (1996)).

Xenogenous porcine SIS has been used recently with favorable results (e.g., Kropp, B.P. et al, *Urology* 46:396 (1995)). This biodegradable collagen-rich xenogenic membrane had been previously studied as a potential material for vascular grafts (see, e.g., Hiles et al., *J. Biomed. Materials Research* 27:139 (1993)). However, SIS may be limited by the maximum size the graft can cover, which may not be sufficient for bladder replacement.

Other problems have been reported with the use of certain gastrointestinal segments for bladder surgery, including infection, perforation, stone formation, metabolic derangements and instances of tumor development. Formalin-preserved sections of bladder have been used for bladder reconstruction (see, e.g., Tsuji et al., *J. Urol*. 98:91 (1967)). However, the use of the formalin-preserved material generally did not result in effective long-term treatment.

Polymeric and naturally-derived "scaffolds" have also been used to support the regrowth of bone into bone defects (see, e.g., U.S. Patent Nos. 5,112,354 and 4,172,128; for a general review, see Yaszemski, M.J.; et al., *Biomaterials* 17 (2): 175-85 (1996) and references cited therein). Bone-derived collagen implants have been used for bone repair. However, these materials do not always provide the requisite strength, flexibility, or non-immunogenicity needed for long-term repair of bone.

### Summary of the Invention

The present invention relates to materials for repairing or augmenting tissues. More particularly, the invention relates to methods for tissue reconstruction or repair using bladder submucosa, to methods for preparing bladder submucosa segments suitable for use in tissue reconstruction or repair, and to materials for use in tissue reconstruction or repair.

In one aspect, the invention provides a composition for use in a method of surgically augmenting a tissue of a subject. The method includes the step of augmenting the tissue of the subject with isolated bladder submucosa. In preferred embodiments, the isolated bladder submucosa comprises isolated bladder submucosa seeded with cells. The cells can be cells of a type found in the tissue of the subject. The tissue of the subject can be bladder tissue. The isolated bladder submucosa can be xenogenic, or, more preferably, allogenic bladder submucosa. The isolated bladder submucosa can further include a growth factor for promoting growth of the tissue. The subject can be a mammal, including a human.

In another embodiment, the invention provides composition for use in a method for repairing a damaged tissue, the method comprising contacting the damaged tissue with isolated bladder submucosa seeded with cells, under conditions such that growth of the tissue occurs, such that the damaged tissue is repaired. The damaged tissue can be bladder tissue.

In another aspect, the invention provides a material for tissue reconstruction or augmentation. The material comprises isolated bladder submucosa seeded with cells. The isolated bladder submucosa can have first and second surfaces, and can be seeded with a first cell type on the first surface and is seeded with a second cell type on the second surface. The first cell type can be urothelial cells and the second cell type can be muscle cells.

In another aspect, the invention provides a method for preparing isolated bladder submucosa seeded with cells. The method includes the steps of seeding the isolated bladder submucosa with cells.

### Brief Description of the Figures

Figure 1 is a schematic diagram showing harvesting of bladder submucosa and cells, and bladder augmentation with the bladder submucosa seeded with cells.

Figure 2 is a chart showing the results of bladder augmentation using isolated bladder submucosa, optionalling including seeded cells.

### Detailed Description of the Invention

The present invention provides methods and materials for tissue reconstruction and repair. In general, the invention features the use of isolated bladder submucosa for tissue repair and augmentation.

Biodegradable polymers have been used as cell delivery vehicles for bladder repair wherein reconstituted muscle cells were layered on one side of the polymer and urothelial cells were layered on the opposite side. The *in vitro* cell-polymer construct was then used for tissue replacement in animal bladders, ureters and urethras (see, e.g., Atala, A. et al., *J. Urol*. 148 (2 Pt 2): 658-62 (1992); Atala, A., et al. *J. Urol*. 150 (2 Pt 2): 608-12 (1993); Yoo, J.J. et al., *J. Urol*. Pt. 2, 153:375A (1995)). Although the polymers were adequate for certain purposes, isolated bladder submucosa has specific characteristics, such as elasticity, which are desirable for use in tissue repair, reconstruction, and augmentation.

Bladder tissue *in vivo* contains three principal layers: the submucosal layer, the muscle layer, and the urothelial layer. As used herein, the term "isolated bladder submucosa" refers to bladder submucosa which is substantially free of naturally-occurring urothelial and muscle layers of bladder. In preferred embodiments, isolated bladder submucosa is substantially free of naturally-occurring adherent muscle and urothelial cells (i.e., isolated bladder submucosa is preferably substantially free of muscle and urothelial cells which were part of the naturally-occurring bladder tissue from which the isolated bladder submucosa was obtained, and which cells were not removed from the submucosa, e.g., by microdissection). In certain embodiments, isolated bladder submucosa is substantially cell-free. Isolated bladder submucosa is a collagen-rich layer which is substantially non-immunogenic, acellular, and bioresorbable.

"Isolated bladder submucosa seeded with cells" refers to isolated bladder submucosa from which substantially all naturally-occurring adherent cells have been removed (as described herein), and to which exogenous cells have been added. The term "exogenous" cells, as used herein, refers to cells which are added *in vitro* to isolated bladder submucosa. Thus, for example, exogenous cells include cells obtained from cell culture or from separate tissue samples. Exogenous cells can be obtained from a sample of whole bladder tissue from which isolated bladder submucosa is obtained; however, such cells must be first separated from the submucosa before the isolated submucosa is seeded with the cells. For example, as described in the Example, *infra*, microdissection of whole bladder tissue can separate the submucosa layer from the urothelial layer and the muscular layer. Cells obtained from the muscle or urothelial tissue can then be cultured *in vitro,* and the cultured cells seeded onto isolated submucosa. Exogenous cells also include cells obtained from organs or tissues other than bladder, such as endothelial cells (e.g., from vascular tissue), osteoblasts from bone, and the like.

Isolated bladder submucosa can be obtained, e.g., according to the methods described herein. For example, sections of bladder harvested from a subject can be microdissected to remove the muscle and urothelial layers from the submucosa (e.g., as described in the Example, *infra*) to produce isolated bladder submucosa, which, in certain embodiments, can be washed, e.g., with phosphate-buffered saline (PBS) to remove extraneous materials, blood, and the like. In certain embodiments, isolated bladder submucosa (e.g., prepared by microdissection) can be further treated to ensure that the isolated bladder submucosa preparation is acellular. For example, sections of microdissected bladder submucosa can be placed in distilled water to lyse any remaining cells which adhere to the collagenous submucosal layer. Further treatments, e.g., with a deoxyribonuclease to remove any remaining nucleic acids, can be employed to further ensure that isolated bladder submucosa is cell-free (prior to any optional seeding with exogenous cells). Such treatments will be routine to one of ordinary skill in the art in light of the teachings herein (see also Sutherland, R.S., et al. *J. Urol*. 156:571 (1996)).

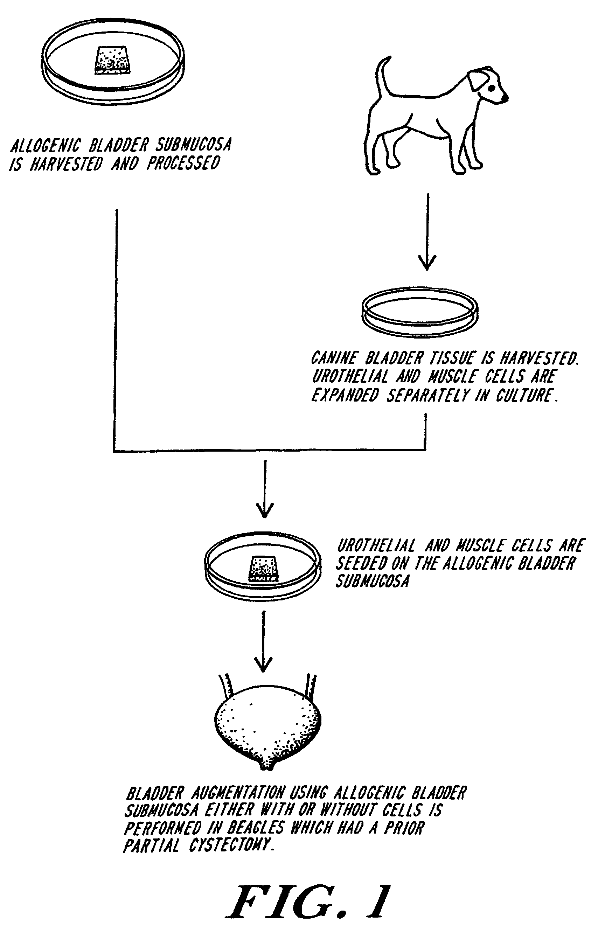

Cells for seeding onto isolated bladder submucosa can be obtained by standard methods and will in general be selected to be compatible with the target tissue or organ which is being repaired or augmented. The seeded cells are preferably of a type which can normally be found in the target organ or tissue. In preferred embodiments, the cells are cells obtained from a donor animal of the same species as the subject (e.g., as shown in Figure 1), to avoid or reduce immunogenic responses in the host after implantation. Such cells are referred to herein as "allogenic" cells. In certain preferred embodiments, the seeded calls can be obtained from the subject (autologous cells) prior to surgery. Cells (such as autologous cells) can be cultured *in vitro,* if desired, to increase the number of cells available for seeding on the isolated bladder submucosa "scaffold". The use of allogenic cells, and more preferably autologous cells, is preferred to prevent tissue rejection. However, if an immunological response does occur in the subject after implantation of the isolated bladder submucosa seeded with cells (which could lead to graft rejection), the subject can be treated, e.g., with immunosuppresive agents such as cyclosporin or FK506, to reduce the likelihood of rejection of the implanted material. In certain embodiments, chimeric cells, or cells from a transgenic animal, can be seeded onto the isolated bladder submucosa.

Seeding of cells onto the isolated bladder submucosa can be performed, e.g., as described in the Example or according to standard methods. For example, the seeding of cells onto polymeric substrates for use in tissue repair has been reported (see, e.g., Atala, A. et al., *J*. *Urol*. 148 (2 Pt 2): 658-62 (1992); Atala, A., et al. *J. Urol*. 150 (2 Pt 2): 608-12 (1993)). In certain preferred embodiments, more than one cell type can be seeded onto isolated bladder submucosa prior to implantation. Illustratively, as described in the Example, *infra*, isolated bladder submucosa can be seeded on one side or surface with urothelial cells, and on a second side or surface with muscle cells, prior to implantation of the graft. In certain embodiments, more than one cell type can be seeded onto a single surface of the isolated bladder submucosa. Cells grown in culture can be trypsinized to separate the cells, and the separated cells can be seeded on the isolated bladder submucosa. Alternatively, cells obtained from cell culture can be lifted from a culture plate as a cell layer, and the cell layer can be directly seeded onto the isolated bladder submucosa without prior separation of the cells. In a preferred embodiment, at least 50 million cells/cm² are seeded onto a surface of isolated bladder submucosa. However, it will be appreciated that the density of cells seeded onto the bladder submucosa can be varied. For example, greater cells densities promote greater tissue formation by the seeded cells, while lesser densities may permit relatively greater formation of tissue by cells infiltrating the graft from the host. Selection of cell types, and seeding of cells onto isolated bladder submucosa, will be routine to one of ordinary skill in the art in light of the teachings herein.

Isolated bladder submucosa can be treated with additives or drugs prior to implantation (before or after the isolated bladder submucosa is seeded with cells, if the optional seeded cells are employed), e.g., to promote the formation of new tissue after implantation. Thus, for example, growth factors, cytokines, extracellular matrix components, and other bioactive materials can be added to the isolated bladder submucosa to promote graft healing and formation of new tissue. Such additives will in general be selected according to the tissue or organ being reconstructed or augmented, to ensure that appropriate new tissue is formed in the engrafted organ or tissue (for examples of such additives for use in promoting bone healing, see, e.g., Kirker-Head, C.A. *Vet. Surg*. 24 (5): 408-19 (1995)). For example, when isolated bladder submucosa (optionally seeded with endothelial cells) is used to augment vascular tissue, vascular endothelial growth factor (VGEF, see, e.g., U.S. Patent No. 5,654,273) can be employed to promote the formation of new vascular tissue. Growth factors and other additives (e.g., epidermal growth factor (EGF), heparin-binding epidermal-like growth factor (HBGF), fibroblast growth factor (FGF), cytokines, genes, proteins, and the like) can be added in amounts in excess of any amount of such growth factors (if any) which may be produced by the cells seeded on the isolated bladder submucosa, if added cells are employed. Such additives are preferably provided in an amount sufficient to promote the formation of new tissue of a type appropriate to the tissue or organ which is to be repaired or augmented (e.g., by causing or accelerating infiltration of host cells into the graft).

While reference is made herein to augmentation of bladder according to the invention, it will be understood that the methods and materials of the invention are useful for tissue reconstruction or augmentation of a variety of tissues and organs in a subject. Thus, for example, organs or tissues such as bladder, ureter, urethra, renal pelvis, and the like, can be augmented or repaired with isolated bladder submucosa seeded with cells. The materials and methods of the invention further can be applied to the reconstruction or augmentation of vascular tissue (see, e.g., Zdrahala, R.J., *J. Biomater. Appl.* 10 (4): 309-29 (1996)), intestinal tissues, stomach, cartilage, bone (see, e.g., Laurencin, C.T. et al., *J. Biomed. Mater. Res.* 30 (2): 133-8 1996), and the like. The term "subject," as used herein, refers to a mammal, such as a dog, cat, pig, horse, cow, or human, in need of reconstruction, repair, or augmentation of a tissue.

Isolated bladder submucosa can be obtained from whole bladder tissue as described herein. Acellular isolated bladder submucosa is believed to be substantially non-immunogenic. In certain preferred embodiments, isolated bladder submucosa for tissue repair or augmentation is obtained from an animal of the same species as the subject; such tissue is referred to herein as "allogenic" bladder submucosa. However, the substantially non-immunogenic qualities of isolated bladder submucosa can permit the use of isolated bladder submucosa obtained from a species different from the subject (referred to herein as "xenogenic" isolated bladder submucosa). The use of xenogenic isolated bladder submucosa is especially advantageous when allogenic isolated bladder submucosa is difficult to obtain, e.g., when the subject is a human. Thus, isolated bladder submucosa can be obtained from animals, such as pigs, from which adequate quantities are readily available, for use in repair or augmentation of tissues or organs of a subject of another species. As previously mentioned, however, allogenic cells are preferred for seeding on the isolated bladder submucosa when cells are employed according to the invention. Additionally, isolated bladder submucosa can be obtained from cadavers.

In a preferred embodiment, the materials of the invention are useful for the reconstruction or augmentation of bladder tissue. Thus, the invention provides treatments for such conditions as bladder exstrophy, bladder volume insufficiency, reconstruction of bladder following partial or total cystectomy, repair of bladder damaged by trauma, and the like.

It has now been found that isolated bladder submucosa, without or without added cells, can permit the formation of new tissue having a grossly normal cellular organization. For example, as described in the Example, *infra*, isolated bladder submucosa, optionally seeded with urothelial and muscle cells and grafted into bladder, served as a "scaffold" for the formation of new bladder tissue within about two months. The new tissue consisted of a urothelial-lined lumen surrounded by submucosal tissue and smooth muscle. Moreover, the newly-formed tissue showed evidence of angiogenesis, and nerve growth. Without wishing to be bound by any theory, it is believed that cells from the host animal can infiltrate and grow on the isolated bladder submucosa graft, thereby providing new tissue which is structurally and functionally similar to native tissue, e.g., bladder tissue. The seeded cells, if employed, may also grow to form new tissue. Isolated bladder submucosa without added cells can, in some instances, be resorbed by the host animal after implantation. It is believed that isolated bladder submucosa seeded with cells is generally not resorbed after implantation. However, in certain instances, the isolated bladder submucosa may be resorbed as new tissue is formed.

Grafting of isolated bladder submucosa, optionally seeded with cells, to an organ or tissue to be augmented can be performed according to the methods described herein or according to art-recognized methods. Thus, for example, isolated bladder submucosa can be grafted to an organ or tissue of the subject by suturing the graft material to the target organ, e.g., as described in Example 1, *infra.* Other methods for attaching a graft to an organ or tissue of the subject (e.g., by use of surgical staples) may also be employed. Such surgical procedures can be performed by one of ordinary skill in the art according to known procedures.

The materials of the invention have been found to be useful in bladder augmentation, as described herein. In a preferred embodiment, isolated bladder submucosa seeded with cells is used for augmentation of bladder, to provide an augmented bladder having a volume (capacity) at least about 20% greater than the pre-augmentation capacity, more preferably at least about 40% greater, 60% greater, 80%, 100%, 200% or 300% greater bladder capacity. In other embodiments, isolated bladder submucosa without cells is used for augmentation of bladder, to provide an augmented bladder having a volume (capacity) at least about 20% greater than the pre-augmentation capacity, more preferably at least about 30% greater, 40% greater, 50%, 70% 100% or 200% greater bladder capacity. It has been found that some contraction of the bladder may occur after grafting of isolated bladder submucosa without cells to bladder. Without wishing to be bound by theory, it is believed that such contraction may be due to self-adherence of the grafted material. In general, it is believed that grafts of isolated bladder submucosa seeded with cells do not significantly contract over time after implantationpossible due to inhibition of self-adherence and contraction by the seeded cells of the grafted material. If desired, the graft site (or the isolated bladder submucosa seeded with cells) can be treated with materials for preventing self-adherence of the graft material, thereby preventing contraction of the grafted bladder and providing increased bladder capacity in the subject. Suitable materials for the prevention of surgical adhesions are known and are commercially available.

### Example

The isolated bladder submucosa, optionally seeded with cells, was prepared as generally depicted in Figure 1. Ten beagles were anesthetized with sodium pentobarbital (25 mg/kg IV) following pretreatment with acepromazine (0.2 mg/kg IM). Beagles underwent partial cystectomies, removing approximately 50% of their bladders. In five, the bladder tissue was microdissected and the mucosal and muscular layers separated. The bladder urothelial and muscle cells were cultured using our previously described technique (see, e.g., Cilento, B.G. et al., *J. Urol*. 152:665 (1994); Tobin, M.S. et al., *Surgical Forum* 45:786 (1994); Freeman, M.R. et al. *J. Urol.* 153:4 (suppl.) (1995)). Briefly, urothelial cells were dissected and placed in serum and free keratinocyte growth medium (Keratinocyte SFM, Gibco, Grand Island, NY) containing 5 ng/mL epidermal growth factor and 50ug/mL bovine pituitary extract. Muscle cells were processed by the tissue explant technique using Dulbecco's Modified Eagle's Medium (DMEM) (HyClone Laboratories, Inc., Logan, Utah) supplemented with 10 % fetal calf serum. The cells were incubated in a humidified atmosphere chamber containing 5% CO₂ and maintained at 37°C.

Canine bladder tissue was aseptically obtained from sacrificed animals. The bladder tissue was repeatedly rinsed with phosphate buffered saline (PBS). The submucosa was microdissected and isolated from the muscular and serosal layers. The isolated submucosa was thoroughly washed and placed in PBS containing 10% cefazolin. The submucosa was then kept at 4° C for 6 to 12 months. All segments of allogenic bladder submucosa, measuring 4 x 5 cm in size, were exposed to UV light for 24 hours to sterilize the segments. Five segments were seeded with the *in vitro* expanded muscle cells on one side and urothelial cells on the opposite side. These cell-submucosa scaffolds were left in culture for 7 days before implantation. The remaining five bladder submucosal segments were not seeded with cells.

Preoperative fluoroscopic cystography and urodynamic studies were performed in all animals. Under general anesthesia, ten beagles underwent cruciate cystotomies on the bladder dome. Augmentation cystoplasty was performed with the allogenic bladder submucosa seeded with urothelial and muscle cells in five animals, and with allogenic isolated bladder submucosa without added cells in the remaining five animals. A single layer of continuous interlocking sutures with 4-0 vicryl was used for anastomosis. 5-0 Nylon nonabsorbable sutures were placed at the four surgical comers as markers. The augmented bladders were covered with omentum. Cystostomy catheters were used for urinary diversion for 10 to 14 days. Urodynamic studies and fluoroscopic cystography were performed in all dogs at one, two and three months post-operatively. The augmented bladders were retrieved two and three months after augmentation and examined grossly and histologically with hematoxylin and eosin stains.

### Results

During the duration of the study, none of the dogs demonstrated any untoward effects. All animals survived until the time of sacrifice without any noticeable complications such as urinary tract infection or calculi formation. Fluoroscopic cystography of all the augmented bladders showed a normal bladder configuration without any leakage at one, two and three months after the procedure.

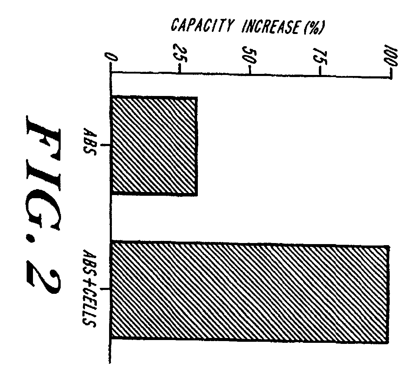

The results are graphically shown in Figure 2 (ABS: allogenic bladder submucosa; ABS + cells: allogenic bladder submucosa seeded with urothelial and muscle cells). Bladders augmented with the allogenic bladder submucosa seeded with cells showed an average increase in capacity of 99%. Bladders augmented with the cell-free isolated bladder submucosa showed an average increase in capacity of 30%. All animals showed a normal bladder compliance as evidenced by the urodynamic studies.

At retrieval, the augmented bladders appeared grossly normal without any evidence of diverticular formation in the region of the graft. The thickness of the grafted segment was similar to that of the native bladder tissue. There was no evidence of adhesion or fibrosis. Histologically, all retrieved bladders contained a normal cellular organization consisting of a urothelial lined lumen surrounded by submucosal tissue and smooth muscle. An angiogenic response was evident in all specimens.

The results show that bladder submucosa seeded with urothelial and muscle cells can form new bladder tissue which is histologically and functionally indistinguishable from the native bladder. This result may be due to a possible maintenance of the architectural frame of the bladder by the extracellular matrix regenerated by the seeded cells. The urothelial and muscle cells seeded on the allogenic submucosa appear to prevent the resorption of the graft. This technology is able to form new bladder tissue which is anatomically and functionally similar to that of normal bladders.

## Claims

1. Isolated bladder submucosa, which submucosa is:
(a) for use in therapy; and/or
(b) seeded with cells (for example of a type found in the tissue of the subject).

2. The submucosa of claim 1 for augmenting (e.g. surgically), repairing or reconstructing tissue in a subject (e.g. a human subject).

3. The submucosa of claim 1 or claim 2 wherein the tissue is bladder tissue.

4. The submucosa of any one of the preceding claims which is allogenic or xenogenic.

5. The submucosa of any one of the preceding claims further comprising a growth factor for promoting growth of the tissue.

6. The submucosa of any one of the preceding claims which has first and second surfaces and is seeded with a first cell type on the first surface and is seeded with a second cell type on the second surface.

7. The submucosa of claim 6, wherein the first cell type comprises urothelial cells and the second cell type comprises muscle cells.

8. An *in vitro* process for producing isolated bladder submucosa seeded with cells comprising the steps of seeding the provided isolated bladder submucosa with cells.

9. Use of isolated bladder submucosa (e.g. as defined in any one of claims 1-7 or as obtainable by the process of claim 8) in the manufacture of a medicament for augmenting (e.g. surgically), repairing or reconstructing tissue in a subject (e.g. a human subject).

## Patentansprüche

1. Isolierte Blasen-Submukosa:
(a) zur Verwendung in der Therapie; und/oder
(b) die mit Zellen beimpft ist (zum Beispiel eines Typs, der im Gewebe des Versuchsobjekts zu finden ist).

2. Submukosa nach Anspruch 1 zum Verstärken (z.B. chirurgisch), Reparieren oder Rekonstruieren von Gewebe in einem Versuchsobjekt (z.B. eine Testperson).

3. Submukosa nach Anspruch 1 oder Anspruch 2, wobei das Gewebe Blasengewebe ist.

4. Submukosa nach einem der vorherigen Ansprüche, die allogen oder xenogen ist.

5. Submukosa nach einem der vorherigen Ansprüche, ferner umfassend einen Wachstumsfaktor zur Wachstumsförderung des Gewebes.

6. Submukosa nach einem der vorherigen Ansprüche, die eine erste und eine zweite Oberfläche hat und die mit einem ersten Zelltyp auf der ersten Oberfläche beimpft ist und mit einem zweiten Zelltyp auf der zweiten Oberfläche beimpft ist.

7. Submukosa nach Anspruch 6, wobei der erste Zelltyp Harnwegsepithelzellen und der zweite Zelltyp Muskelzellen umfasst.

8. *In-vitro*-Verfahren zur Herstellung isolierter Blasen-Submukosa, die mit Zellen beimpft ist, umfassend das Beimpfen der bereitgestellten isolierten Blasen-Submukosa mit Zellen.

9. Verwendung isolierter Blasen-Submukosa (z.B. gemäß einem der Ansprüche 1-7 oder herstellbar mit dem Verfahren nach Anspruch 8) zur Herstellung eines Medikamentes zum Verstärken (z.B. chirurgisch), Reparieren oder Rekonstruieren von Gewebe in einem Versuchsobjekt (z.B. einer Testperson).

## Revendications

1. Sous-muqueuse vésicale isolée, ladite sous-muqueuse étant :
(a) prévue pour être utilisée en thérapie ; et/ou
(b) ensemencée de cellules (par exemple d'un type trouvé dans le tissu du sujet).

2. La sous-muqueuse de la revendication 1 pour augmenter (par exemple par voie chirurgicale), réparer ou reconstruire un tissu chez un sujet (par exemple un sujet humain).

3. La sous-muqueuse de la revendication 1 ou de la revendication 2, dans laquelle le tissu est du tissu vésical.

4. La sous-muqueuse de l'une quelconque des revendications précédentes, qui est allogène ou xénogène.

5. La sous-muqueuse de l'une quelconque des revendications précédentes comprenant en outre un facteur de croissance pour promouvoir la croissance du tissu.

6. La sous-muqueuse de l'une quelconque des revendications précédentes, qui a des première et deuxième surfaces et est ensemencée avec un premier type de cellule sur la première surface et ensemencée avec un deuxième type de cellule sur la deuxième surface.

7. La sous-muqueuse de la revendication 6, dans laquelle le premier type de cellule comprend des cellules urothéliales et le deuxième type de cellule comprend des cellules musculaires.

8. Procédé *in vitro* pour produire une sous-muqueuse vésicale isolée avec des cellules comprenant l'étape d'ensemencement de la sous-muqueuse vésicale isolée fournie avec des cellules.

9. Utilisation d'une sous-muqueuse vésicale isolée (par ex. telle que celle définie dans l'une quelconque des revendications 1-7 ou pouvant être obtenue par le procédé de la revendication 8) dans la fabrication d'un médicament pour augmenter (par ex. par voie chirurgicale), réparer ou reconstruire le tissu chez un sujet (par ex. un sujet humain).
